# EUROPÄISCHE PATENTSCHRIFT

(11) **EP 0 297 389 B1**
(45) Veröffentlichungstag und Bekanntmachung des Hinweises auf die Patenterteilung: **10.03.1993**
(21) Anmeldenummer: 88109827.1
(22) Anmeldetag: 21.06.1988
(51) Int. Cl.: G01N 33/52, G01N 31/22

(54) **Diagnostischer Testträger und Verfahren zu dessen Herstellung**
Diagnostic-test support and process for its manufacture
Support de test diagnostique et procédé pour sa fabrication

(30) Priorität: 27.06.1987 DE 3721237
(43) Veröffentlichungstag der Anmeldung: 04.01.1989
(73) Patentinhaber: BOEHRINGER MANNHEIM GMBH, 68298 Mannheim (DE)
(72) Erfinder: Macho, Heinz Kurt, D-6149 Fürth/Fahrenbach (DE); Hungenberg, Klaus Dieter, Dipl.Chem.Dr., D-6943 Birkenau-Hornbach (DE); Becker, Norbert, D-6840 Lampertheim (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 164 960
- EP-A- 0 287 883
- EP-A- 0 290 921
- AT-B- 0 360 961
- DE-A- 2 641 097
- US-A- 4 258 001
- US-A- 4 426 451
- D.L. Bateman, "Hot Melt Adhesives", Noyes Data Corp., 1978, S. 1;
- E.L. Crowell et al. "Hot Melt Adhesives" aus "Handbook of Adhesives", Robert E. Krieger Publishing Company, S. 447;

## Beschreibung

Die Erfindung betrifft einen diagnostischen Testträger mit mindestens zwei Schichten, die mit Abstand zueinander befestigt sind, so daß ein Spalt zwischen ihnen vorhanden ist. Weiter richtet sie sich auf ein Verfahren zur Herstellung eines solchen Testträgers.

Zur qualitativen oder quantitativen analytischen Bestimmung von Bestandteilen von Körperflüssigkeiten, insbesondere von Blut, werden in jüngerer Zeit zunehmend sogenannte trägergebundene Tests verwendet. Bei diesen sind Reagenzien in entsprechenden Schichten eines festen Testträgers eingebettet, der mit der Probe in Kontakt gebracht wird. Die Reaktion von Probe und Reagenzien führt zu einem nachweisbaren Signal, insbesondere einem Farbumschlag, welcher visuell oder mit Hilfe eines Gerätes, meistens reflexionsphotometrisch, ausgewertet werden kann.

Testträger sind häufig als Teststreifen ausgebildet, die im wesentlichen aus einer länglichen Tragschicht aus Kunststoffmaterial und darauf angebrachten Testfeldern bestehen. Es sind jedoch auch Testträger bekannt, die als quadratische oder rechteckige Plättchen gestaltet sind.

Testträger der eingangs bezeichneten Art sind beispielsweise aus Research Disclosure, Vol. 200 (1980), Abstrakt 22, Seite 554 bis 557 (in folgenden als RD zitiert) bekannt. Dort werden die Schichten als Unterteil (support member) bzw. als Deckelteil (cover member) bezeichnet. Beide Teile sind flache Scheiben aus einem starren Kunststoffmaterial. Zur Erzeugung des Spaltes ist zwischen den Teilen an ihrem Umfang ein Zwischenstück flüssigkeitsdicht eingesetzt, wobei die Verbindung durch konventionelle Klebstoffe oder durch Ultraschallschweißen erzeugt sein kann.

Eine ähnliche Testträgerkonstruktion wird in dem US-Patent 4 426 451 beschrieben.

Aus der DE-A-2641097 bzw. der US-A-4088 448 ist eine testträgerförmige Küvette bekannt, bei der ein Spalt innerhalb eines einstückigen Kunststoffteils vorhanden ist.

In der EP-A-0 287 883, welche einen nicht vorveröffentlichten Stand der Technik im Sinne von A 54 (3) bildet, ist ein Analyseelement in Form eines Teststreifens beschrieben, bei dem die Zuführung eines definierten Probevolumens zu einem Reagenzfeld dadurch erreicht werden soll, daß sich darunter ein Spalt befindet. Der zur Sicherstellung des Spaltes erforderliche Abstand wird durch eine Abstandhalterschicht gewährleistet, deren Teilbereiche sich an der äußeren Begrenzung des Testfeldes entlang erstrecken, so daß sie jeweils einen einzigen Zwischenraum einschließen. Die Abstandshalterschicht kann erhitzt und dadurch benutzt werden, um das Reagenzfeld anzuhaften.

In allen diesen Fällen dient der Spalt dazu, mit Hilfe der Kapillarkraft eine Flüssigkeit in dem Spalt zu transportieren. Dies ist bei der Konstruktion von diagnostischen Testträgern vielfach ein nützliches Konstruktionselement. Beispielsweise kann der Spalt, wie in der Literaturstelle RD beschrieben, dazu dienen, einen bestimmten Hohlraum des Testträgers mit einer Probenflüssigkeit zu füllen. Gegebenenfalls kann, wie ebenfalls in RD beschrieben wird, der Flüssigkeitsstrom auch unterbrochen und aufgrund eines Druckstoßes wieder fortgesetzt werden.

Trotz dieser Vorteile haben sich Spaltkonstruktionen für Testträger bislang nicht in großem Umfang durchsetzen können, was darauf zurückzuführen sein dürfte, daß die vorbekannten Testträger dieser Art aufwendig in der Herstellung sind.

Aufgabe der vorliegenden Erfindung ist es daher, einen diagnostischen Testträger und ein Verfahren zu dessen Herstellung zur Verfügung zu stellen, bei dem ein einen Flüssigkeitstransport zwischen zwei Schichten des Testträgers ermöglichender Spalt vorhanden ist, wobei der Testträger einfach und kostengünstig in großen Stückzahlen herstellbar sein soll.

Der erfindungsgemäße diagnostische Testträger zeichnet sich durch die Merkmale des Anspruchs 1 aus.

Das erfindungsgemäße Verfahren zeichnet sich durch die Merkmale des Anspruchs 7 aus.

Die durch die Schmelzkleberbereiche verbundenen Schichten können praktisch aus sämtlichen Materialien bestellen, die für die Herstellung mehrschichtiger Testträger üblicherweise verwendet werden. In Frage kommen insbesondere nichtsaugende Materialien, hauptsächlich Kunststoffolien. Es können aber auch Schichten aus saugfähigen Materialien, wie beispielsweise Papiere, Vliese, Gewebe, Gewirke oder poröse Kunststoffmaterialien als Schichtmaterialien eingesetzt werden. Vorzugsweise besteht jedoch mindestens eine der Schichten, die den Spalt begrenzen, aus einem nicht saugfähigen Material.

Als Schmelzkleber können handelsübliche Produkte, beispielsweise auf Basis von Ethylen-Vinylacetat-Copolymeren, Polyestern oder Polyamiden zum Einsatz kommen. Derartige Schmelzkleber wurden auch bisher schon bei der Herstellung von diagnostischen Testträgern verwendet, wobei sie üblicherweise vollflächig auf die eine zu verbindende Schicht aufgetragen werden und die andere Schicht dann gegen die Schmelzkleberfläche gepreßt wurde.

Bei einem anderen bekannten Verfahren wird ein Streifen von Schmelzklebermaterial verwendet, um eine oder mehrere Testträgerschichten an ihrer Kante mit einer Unterlage zu verbinden.

Überraschend wurde nun im Rahmen der vorliegenden Erfindung gefunden, daß ein reproduzierbarer und gleichmäßiger Spalt (insbesondere zum Zwecke des Flüssigkeitstransport) in einem Testträger gebildet werden kann, wenn man den Schmelzkleber nicht vollflächig, sondern lediglich bereichsweise auf das eine zu verbindende Schichtmaterial aufträgt, wobei dei Dicke des Schmelzkleberauftrags größer als die gewünschte Spaltbreite ist, und das zweite Schichtmaterial mit Hilfe geeigneter Maßnahmen, die weiter unten noch näher erläutert werden, nicht fest dagegenpreßt, sondern nur so andrückt, daß ein Abstand entsprechend der gewünschten Spaltbreite bestehen bleibt.

Bei dem Andrücken muß der Schmelzkleber ausreichend heiß sein, daß er mit dem zweitem Schichtmaterial bindet. Bevorzugt wird dies dadurch erreicht, daß das zweite Material kurzfristig nach dem Schmelzkleberauftrag, also während der sogenannten "offenen Zeit" des Schmelzklebers aufgetragen wird. In Ausnahmefällen, beispielsweise wenn das erste Schichtmaterial nach dem Auftragen der Schmelzkleberbereiche mit einem Netzmittel beschichtet werden soll, kann es jedoch auch zweckmäßig sein, den Schmelzkleber nach dem Auftragen der Bereiche zunächst erstarren zu lassen und vor Aufbringen des zweiten Materials erneut zu erhitzen.

Die Schmelzkleberbereiche können sehr verschiedene Formen haben. Sie müssen jedenfalls so gestaltet sein, daß ausreichend große Zwischenräume bleiben, daß ein Flüssigkeitstransport zwischen ihnen hindurch möglich ist. Bevorzugt bedecken sie zwischen 10% und 70%, besonders bevorzugt zwischen 15% und 50% der gesamten Verbindungsfläche der Schichten.

Die Erfindung wird im folgenden anhand von in den Figuren schematisch dargestellten Ausführungsbeispielen näher erläutert. Es zeigen:
- Fig. 1: Einen erfindungsgemäßen Testträger in perspektivischer Darstellung;
- Fig. 2: Einen Querschnitt durch den Testträger gemäß Fig. 1 entlang der Linie II-II;
- Fig. 3: Eine Aufsicht auf ein Stück Schichtmaterial mit Schmelzkleberbereichen in Form von Streifen;
- Fig. 4: Eine Aufsicht entsprechend Fig. 3 mit Schmelzkleberbereichen in Form runder Partikel;
- Fig. 5: eine Prinzipdarstellung eines Herstellungsverfahrens mit Hilfe von Siedruck;
- Fig. 6: Eine Prinzipdarstellung eines Herstellungsverfahrens mit Radauftrag.

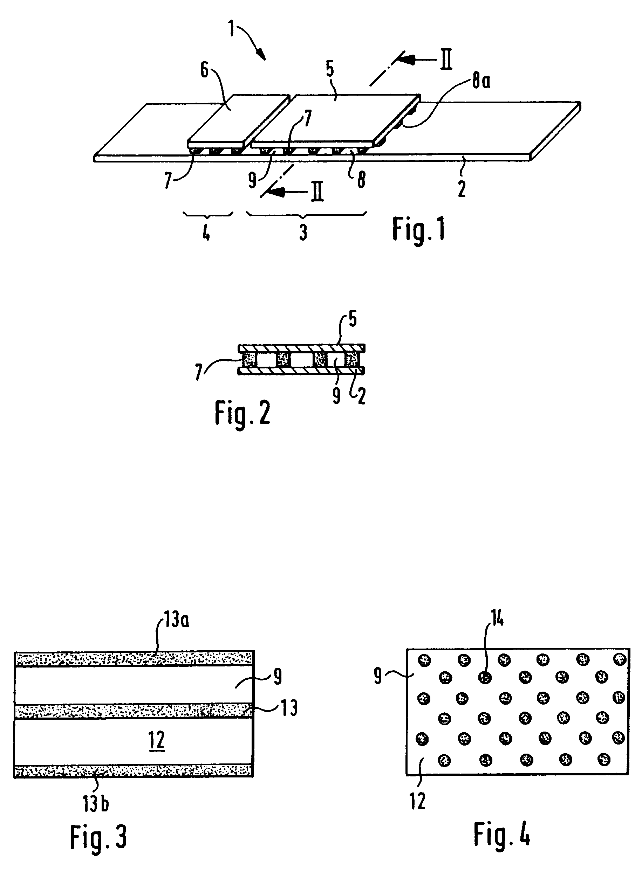

Der in den Figuren 1 und 2 dargestellte Testträger 1 weist eine Tragschicht 2 auf, auf welcher unmittelbar nebeneinander eine Flüssigkeitstransportzone 3 und eine Nachweiszone 4 angeordnet sind.

Im Bereich der Flüssigkeitstransportzone 3 ist eine nichtsaugende Deckschicht 5 und im Bereich der Nachweiszone 4 ist eine saugfähige Nachweisschicht 6 jeweils mit Schmelzkleberbereichen 7 an der Tragschicht 2 dergestalt befestigt, daß ein Spalt 8 zwischen der Tragschicht 2 und den Schichten 5 bzw. 6 vorhanden ist.

In dem Spalt 8 kann eine Probenflüssigkeit durch Kapillarkraft transportiert werden. Wird sie beispielsweise dem von der Nachweiszone 4 abgewandtem Ende 8a des Spaltes 8 zugeführt, so breitet sie sich zunächst zwischen der Deckschicht 5 und der Tragschicht 2 im Bereich der Flüssigkeitstransportzone 3 aus, wobei sie durch die in Figur 2 besser zu erkennenden Zwischenräume 9 zwischen den Schmelzkleberbereichen hindurchströmt. Durch die Verbindung von zwei nichtsaugenden Materialien mit einem dazwischen liegenden Spalt läßt sich also aus einfache Weise eine Flüssigkeitstransportstrecke erzeugen. Auch bei Großserienherstellung erhält man mit ausgezeichneter Reproduzierbarkeit ein gewünschtes Ausbreitungsverhalten der Flüssigkeit, ohne daß dieses, wie bei Verwendung kapillaraktiver Flüssigkeitstransportschichten (z.B. Vliese, Gewebe) von Chargenschwankungen des Materials abhängig ist.

Bei weiterer Zufuhr von Flüssigkeit gelangt die Probenflüssigkeit auch in den Spalt zwischen Nachweisschicht 6 und Tragschicht 2 im Nachweisbereich 4. Dabei benetzt sie die Nachweisschicht 6 von ihrer Unterseite her, so daß die Probenflüssigkeit in die saugfähige Nachweisschicht 6 eindringt und mit darin befindlichen Reagenzien reagieren kann. Dies kann beispielsweise zu einem für die gewünschte analytische Bestimmung charaktertistischen Farbumschlag führen. Durch die erfindungsgemäße Verbindung einer nichtsaugenden Schicht mit einer saugenden Schicht läßt sich also ein Transport zunächst in Schichtrichtung zur Verteilung der Probenflüssigkeit und eine anschließende Penetration derselben in die saugende Schicht realisieren.

Einzelheiten der Reagenzzusammensetzung oder der Analysemethoden sind nicht Gegenstand der vorliegenden Erfindung. Vielmehr kann eine Vielzahl von bekannten Testträgerreaktionen eingesetzt werden.

Ein Testträger mit dem erfindungsgemäßen Aufbau zweier benachbarter, durch einen Spalt voneinander getrennter Schichten kann auch konstruktiv in sehr verschiedener Weise aufgebaut sein. Figur 1 ist insoweit lediglich als Beispiel für eine mögliche Nutzanwendung zu verstehen, wobei der dargestellte Testträger durch bekannte ergänzende Elemente dem jeweils gewünschten Testablauf angepaßt werden kann.

So kann beispielsweise dem Ende 8a des Kapillarspaltes 8 einfach ein Blutstropfen zugeführt werden, der dann in den Kapillarspalt aufgesaugt wird. Dem Ende 8a können aber auch andere Testträgerschichten vorgelagert sein, durch die die Probe in den Kapillarspalt 8 gelangt, wobei sie beim Durchgang durch die vorgelagerten Schichten beispielsweise Reagenzien löst und eine Vorreaktion stattfindet.

Insgesamt wird deutlich, daß ein zwischen zwei Testträgerschichten befindlicher Kapillarspalt, durch den eine Probenflüssigkeit strömen kann, in vielerlei Weise in der Testträger-Technologie eingesetzt werden kann.

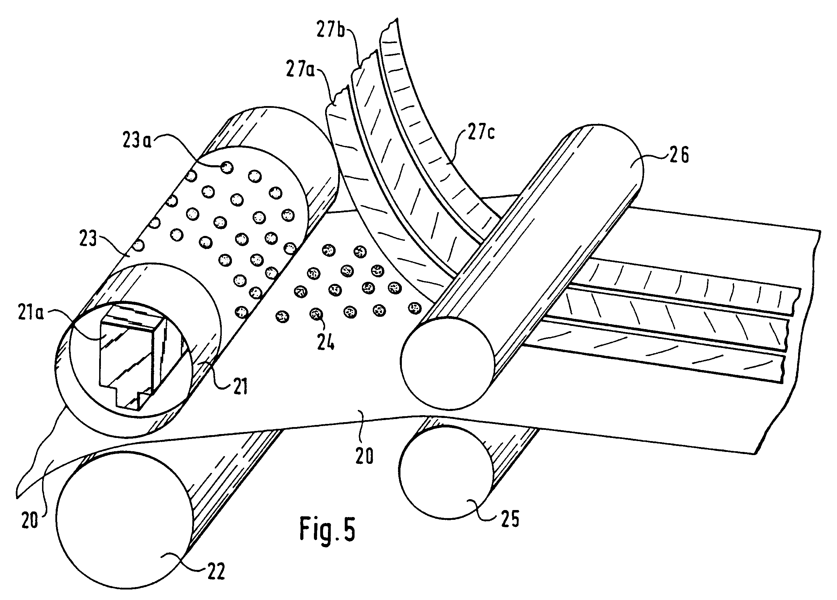

Die Figuren 3 und 4 zeigen beispielhaft zwei verschiedene mögliche Gestaltungen der Schmelzkleberbereiche.

In Figur 3 haben die auf dem Schichtmaterial 12 aufgetragenen Schmelzkleberbereiche die Form von Streifen 13. Sie können, wie dargestellt in Längsrichtung eines Testträgers verlaufen. Dabei kann es zweckmäßig sein, daß mindestens ein Teil der Streifen 13a und 13b nahe den Rändern der zu verklebenden Schichten angeordnet sind, so daß der dazwischenliegende Hohlraum zur Seite des Testträgers hin abgeschlossen wird.

Soweit derartige streifenförmige Schmelzkleberbereiche verwendet werden, ist es erforderlich, daß die Zwischenräume 9 in der gewünschten Strömungsrichtung der zu transportierenden Probenflüssigkeit verlaufen. Außerdem muß auf eine relativ genaue Anordnung der Schmelzkleberstreifen 13 auf dem Schichtmaterial 12 geachtet werden.

Überraschenderweise hat sich gezeigt, daß sehr gute Ergebnisse erreicht werden, wenn der Schmelzkleber in Form von Partikeln, also beispielsweise in Form von runden, quadratischen oder vieleckigen Punkten auf das erste Schichtmaterial aufgetragen wird. In Figur 4 sind beispielhaft runde Punkte 14 dargestellt. Bei einem derartigen Schmelzkleberauftrag sind keine besonderen Justierungsmaßnahmen erforderlich. Dennoch wird ein problemloser Flüssigkeitstransport erreicht. Dies gilt selbst dann, wenn in einem streifenförmigen Testträger eine verhältnismäßig große Flüssigkeitstransportstrecke realisiert werden soll, wie beispielsweise bei dem in der deutschen Patentanmeldung 36 43 516,auf welche hier Bezug genommen wird,beschriebenen Testträger. Obwohl die Seitenkanten der Flüssigkeitstransportstrecke bei partikelförmigem Schmelzkleberauftrag offen bleiben, wird ein zuverlässiger und vollständiger Flüssigkeitstransport erreicht. Dies erstaunt um so mehr, als die für Schmelzkleber verwendeten Polymere hydrophob sind.

Um einen möglichst gleichmäßigen Spaltabstand zwischen den beiden durch die Schmelzkleberbereiche verbundenen Schichten sicherzustellen, sind die Schmelzkleberbereiche bevorzugt gleich groß und gleichmäßig über die gesamte Verbindungsfläche verteilt. In besonderern Fällen kann es jedoch zweckmäßig sein, von dieser Regel abzuweichen. Wenn beispielsweise in einem Testträger eine optische Auswertung der zwischen den Schichten vorhandenen Flüssigkeit notwendig ist, muß mindestens eine der Schichten durchsichtig sein.

In diesem Fall könnten Schmelzkleberpartikel in dem für die optische Auswertung vorgesehenen Bereich stören. Praktische Experimente haben ergeben, daß ein gleichmäßiger Abstand zwischen den Schichten auch dann noch realisierbar ist, wenn aufgrund solcher Erfordernisse die Schmelzkleberbereiche nicht gleichmäßig verteilt sein können. In diesem Fall ist es zweckmäßig, Schichtmaterialien zu verwenden, die eine ausreichende Steifigkeit haben.

Bevorzugt haben die Schmelzkleberpartikel eine durchschnittliche Grundfläche zwischen 0,03 mm² und 5,0 mm². Besonders bevorzugt beträgt die Obergrenze 0,1 mm².

Die Dicke der Schmelzkleberbereiche, die für die Breite des Spaltes bestimmend ist, liegt bevorzugt zwischen 0,02 mm und 1,0 mm, besonders bevorzugt zwischen 0,02 mm und 0,3 mm. Bei Einhaltung dieser bevorzugten Werte ergeben sich besonders vorteilhafte Flüssigkeitstransporteigenschaften.

Figur 5 zeigt in schematisierter perspektivischer Ansicht ein erfindungsgemäßes Herstellungsverfahren, bei dem die Schmelzkleberbereiche im Siebdruckverfahren aufgebracht werden.

Ein erstes Schichtmaterial 20 wird zwischen einer Siebwalze 21 und einer Beschichtungswalze 22, welche vorzugsweise heizbar oder auch kühlbar ist, hindurch transportiert. Dabei wird Schmelzkleber mittels einer Breitschlitzdüse 21a durch die Löcher 23a des Siebes 23 hindurchgedrückt, wobei die Form der dabei erzeugten Schmelzkleberpartikel 24 durch die Form der Löcher 23a des Siebes 23 bestimmt ist.

Das Schichtmaterial 20 mit den Schmelzkleberpartikeln 24 wird weiter transportiert durch einen Spalt zwischen einer unteren Andruckwalze 25 und einer oberen Andruckwalze 26. Dem gleichen Spalt werden in der dargestellten Ausführungsform drei Bahnen 27a, 27b, 27c eines zweiten Schichtmaterials zugeführt, wobei gegebenenfalls auch verschiedene zweite Schichtmaterialien in einem Arbeitsgang mit dem ersten Schichtmaterial verbunden werden können. Es kann vorteilhaft sein, das zweite Schichtmaterial auf der dem ersten Schichtmaterial zugewandten Seite mit einer ein Netzmittel enthaltenden Schicht zu versehen.

Wesentlich für die Erfindung ist nun, daß das zweite Schichtmaterial 27a, 27b, 27c nicht so fest durch die Walzen 25 und 26 zusammengepreßt wird, daß kein Abstand mehr zwischen den Schichtmaterialien verbleibt oder die Schmelzkleberpartikel 24 so stark auseinandergedrückt werden, daß die zwischen ihnen bestehenden Zwischenräume verschwinden, also eine durchgängige Schmelzkleberschicht entsteht. Vielmehr muß das Andrücken so erfolgen, daß ein definierter und reproduzierbarer Abstand zwischen den Schichtmaterialien erhalten bleibt.

Dies wird bevorzugt dadurch erreicht, daß die Andruckwalzen 25, 26 auf einen festen Abstand eingestellt sind. Es kann jedoch auch zweckmäßig sein, den Anpreßdruck zwischen den Walzen 25 und 26 so einzustellen, daß unter Berücksichtigung der Materialeigenschaften der Schichtmaterialien und der Zähigkeit der Schmelzkleberpartikel 24 der gewünschte Abstand erreicht wird.

Die Transportgeschwindigkeit des Schichtmaterials 20 und der Abstand zwischen der Siebwalze 21 und den Andruckwalzen 25, 26 bestimmen die Zeitdauer zwischen dem Aufbringen des Schmelzklebers und dem Andrücken des zweiten Schichtmaterials. Es ist wichtig, daß diese Zeit so bemessen ist, daß der Schmelzkleber noch ausreichen heiß ist, um bindefähig zu sein, wobei er aber andererseits bevorzugt schon so weit abgekühlt ist, daß er eine relativ hohe Viskosität hat, die den Schmelzkleberpartikeln 24 eine ausreichende Stabilität gibt.

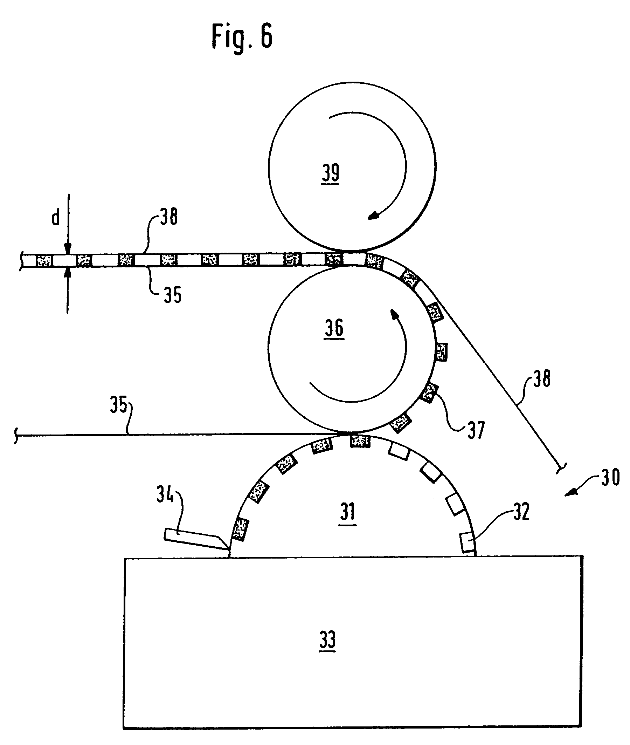

In Figur 6 ist eine andere Möglichkeit dargestellt, die Schmelzkleberbereiche aufzutragen, nämlich mit einem Radauftragswerk 30.

Dessen Auftragsrad 31 hat an seiner Oberfläche eine Vielzahl von Vertiefungen 32, die in Querschnitt und Tiefe den gewünschten Schmelzkleberbereichen entsprechen. Sie werden dadurch mit Schmelzkleber gefüllt, daß sich das Rad 31 in einem schmelzklebergefüllten Behälter 33 dreht, wobei ein Überschuß an Schmelzkleber durch ein Abstreiflineal 34 abgestreift wird.

Ein erstes Trägermaterial 35 wird zwischen dem Auftragsrad 31 und einer Umlenkwalze 36 hindurchgeführt, wobei Schmelzkleberpartikel 37 aufgebracht werden. Das zweite Schichtmaterial 38 wird durch die Druckwalze 39 analog zu der Ausführungsform gemäß Figur 5 dergestalt gegen die Schmelzkleberpartikel 37 gedrückt, daß ein in der Figur mit d bezeichneter Abstand zwischen den Schichten des fertigen Verbundmaterials erhalten bleibt.

Das zweite Schichtmaterial wird schräg von unter dergestalt zugeführt, daß es die Umlenkwalze 36 teilweise umschlingt. Dadurch wird eine besonders gute Verbindung erreicht. Der Anpreßdruck und damit die Größe des Abstandes d bei einer gegebenen Schmelzkleberauftragsstärke kann in diesem Fall auch durch die Spannung kontrolliert werden, unter der das zweite Schichtmaterial 38 zu- bzw. abgeführt wird.

Eine nähere Darstellung der Auftragsverfahren ist nicht notwendig, da der Auftrag von Schmelzkleber sowohl im Siebdruckverfahren als auch der Radauftrag für andere Anwendungszwecke bekannt sind.

Die Erfindung wird im folgenden anhand einiger Beispiele näher erläutert.

### Beispiel 1

Verbindung von nichtsaugenden Materialien.

Mit einem Radauftragsverfahren gemäß Figur 6 wird EVA-Schmelzkleber vom Typ Jet-Melt 3764 der 3M Company mit einer Temperatur von 170° C aufgetragen. die Schmelzkleberpartikel haben dabei eine Grundfläche von 1,1 x 0,5 mm. Der Andruck erfolgt über eine Druckwalze, die auf 70° C temperiert ist, wobei ein fester Spalt zwischen den zu verbindenden Schichten von 70 µm Breite eingestellt ist.

Die erste Schicht, auf die die Schmelzkleberpartikel aufgetragen werden, ist eine Polyethylenterephatalatfolie von 0,35 mm Stärke. Sie wird mit Hilfe der Schmelzkleberpartikel mit einer Polycarbonatfolie von 0,25 mm Stärke verbunden.

Die Schmelzkleberpartikel werden mit einer solchen Dichte gleichmäßig aufgebracht, daß insgesamt etwa 26% der Verbindungsfläche mit Schmelzkleber bedeckt sind.

Der so erhaltende Schichtverbund eignet sich ausgezeichnet als Kapillartransportstrecke innerhalb eines Testträgeraufbaus. Beispielsweise füllt sich ein Probeabschnitt von 30 mm Länge und 5 mm Breite innerhalb von 10 sec gleichmäßig mit einer versuchsweise verwendeten marlonhaltigen Glucoselösung, wobei etwa 15 µ l Flüssigkeit aufgenommen werden.

### Beispiel 2

Verwendet man statt der Polycarbonatfolie in Beispiel 1 eine Gel-Bond-Folie der FMC-Corporation, so ergibt sich eine schnellere Füllgeschwindigkeit des Kapillarspaltes. Er wird mit marlonhaltiger Glucoselösung in etwa 4 sec. gefüllt (mit Blut beträgt die Füllzeit etwa 25 sec.).

### Beispiel 3

Verbindung eines saugenden und eines nichtsaugenden Materials.

Mit einem Radauftragswerk wird eine Polystyrolfolie mit einem multifilen Polyestergewebe, welches eine Reagenzbeschichtung trägt, verbunden.

Die aufgetragenen Schmelzkleberpartikel haben einen Durchmesser von ca. 0,4 mm bis 0,5 mm und bedecken die Verbindungsfläche zu ca. 55%.

Verwendet wird ein EVA-Schmelzkleber vom Typ Jet-Melt 3764 der 3M Company, welcher mit einer Auftragstemperatur von 150° C aufgetragen wird. Die Druckwalze ist auf eine Temperatur von 40° C temperiert.

Bei diesem Beispiel wird die Kapillarspaltbreite dadurch variiert, daß der Anpreßdruck, mit dem die beiden Schichten gegeneinandergedrückt werden, variiert wird.

Für eine Probenfläche von ca. 10 mm x 6 mm ergeben sich folgende Ergebnisse:

Bei einem Anpreßdruck von 0,4 bar füllt sich der Spalt in 6,5 sec mit insgesamt ca. 9 mg Blut.

Bei einem Anpreßdruck von 0,5 bar füllt sich der Spalt in 9,5 sec mit ca. 8,2 mg Blut.

Bei einem Anpreßdruck von 0,8 bar füllt sich der Spalt in 13 sec mit ca. 6,7 mg Blut.

## Patentansprüche

1. Diagnostischer Testträger mit mindestens zwei Schichten (2,5;2,6), die mit Abstand zueinander befestigt sind, so daß ein Spalt (8) zwischen ihnen vorhanden ist,
**dadurch gekennzeichnet,** daß
die Schichten (2,5;2,6) durch eine Mehrzahl diskreter Schmelzkleberbereiche (7,13,14) miteinander verbunden sind, wobei
die Dicke der Schmelzkleberbereiche (7,13,14) fur die Breite des Spaltes (8) bestimmend ist,
die Schmelzkleberbereiche auf der Verbindungsfläche zwischen den Schichten (2,5;2,6) verteilt sind,
zwischen den Schmelzkleberbereichen mehrere Zwischenräume (9) vorhanden sind, und
ein Flüssigkeitstransport in dem Spalt (8) durch die Zwischenräume (9) zwischen den Schmelzkleberbereichen (7,13,14) hindurch möglich ist.

2. Diagnostischer Testträger nach Anspruch 1, **dadurch gekennzeichnet,** daß die Schmelzkleberbereiche (7,13,14) eine Dicke von 0,02 bis 1,0 mm, vorzugsweise von 0,02 bis 0,3 mm haben.

3. Testträger nach Anspruch 1, **dadurch gekennzeichnet,** daß die Bereiche Partikel (7,14) sind.

4. Testträger nach Anspruch 3, **dadurch gekennzeichnet,** daß die Partikel (7,14) eine durchschnittliche Grundfläche zwischen 0,03 mm² und 5,0 mm² haben.

5. Testträger nach Anspruch 1, **dadurch gekennzeichnet,** daß die Gesamtfläche der Schmelzkleberbereiche (7,13,14) zwischen 10% und 70%, vorzugsweise zwischen 15% und 50% der gesamten Verbindungsfläche der Schichten beträgt.

6. Testträger nach Anspruch 1, **dadurch gekennzeichnet,** daß er eine Tragschicht (2) und mehrere darauf nebeneinander angeordnete Zonen (3,4) aufweist und daß mindestens eine der Zonen eine Flüssigkeitstransportzone (3) ist, in der beide durch die Schmelzkleberbereiche (7) miteinander verbundenen Schichten (2,5) nicht saugfähig sind und daß mindestens eine weitere Zone eine Auswertezone (4) ist, in der mindestens eine der miteinander durch Schmelzkleberbereiche (7) verbundenen Schichten (6) saugfähig ist.

7. Verfahren zur Herstellung eines Testträgers nach einem der Ansprüche 1 - 6, **dadurch gekennzeichnet,** daß der Schmelzkleber in Form der diskreten Bereiche zwischen denen Zwischenräume vorhanden sind, auf ein erstes Schichtmaterial in einer Stärke, die größer als der gewünschte Spalt ist, aufgebracht wird und ein zweites Schichtmaterial, während der Schmelzkleber ausreichend heiß ist, um bindefähig zu sein, derartig gegen die Schmelzkleberbereiche gedrückt wird, daß es mit dem Schmelzkleber bindet, jedoch ein durch die Dicke der erstarrten Schmelzkleberbereiche bestimmter Abstand zwischen den Schichtmaterialien bestehen bleibt.

8. Verfahren nach Anspruch 7, **dadurch gekennzeichnet,** daß das Andrücken des zweiten Schichtmaterials so kurz nach dem Auftragen der Schmelzkleberbereiche auf das erste Schichtmaterial erfolgt, daß diese noch ausreichend heiß sind um bindefähig zu sein.

9. Verfahren nach Anspruch 8, **dadurch gekennzeichnet,** daß das zweite Schichtmaterial mit einer ein Netzmittel enthaltenden Schicht versehen ist.

10. Verfahren nach Anspruch 7, **dadurch gekennzeichnet,** daß das Andrücken des zweiten Schichtmaterials mit Hilfe von zwei Walzen erfolgt, zwischen denen die Schictmaterialien hindurchlaufen, wobei der Abstand durch den Abstand der Walzen bestimmt wird.

11. Verfahren nach Anspruch 7, **dadurch gekennzeichnet,** daß die Schmelzkleberbereiche im Siebdruckverfahren aufgebracht werden.

12. Verfahren nach Anspruch 7, **dadurch gekennzeichnet,** daß die Schmelzkleberbereiche mit einem Radauftragswerk aufgebracht werden.

## Claims

1. Diagnostic test carrier with at least two layers (2,5; 2,6) which are fixed together at a distance so that a gap (8) is present between them, characterised in that the layers (2,5; 2,6) are connected with one another by a plurality of discrete melt adhesive regions (7, 13, 14), whereby the thickness of the melt adhesive regions (7, 13, 14) determines the gauge of the gap (8), the melt adhesive regions are distributed on the connecting surface between the layers (2,5; 2,6), several intermediate spaces (9) are present between the melt adhesive regions and a liquid transport in the gap (8) is possible through the intermediate spaces (9) between the melt adhesive regions (7, 13, 14).

2. Diagnostic test carrier according to claim 1, characterised in that the melt adhesive regions (7, 13, 14) have a thickness of 0.02 to 1.0 mm, preferably of 0.02 to 0.3 mm.

3. Test carrier according to claim 1, characterised in that the regions are particles (7, 14).

4. Test carrier according to claim 3, characterised in that the particles 7, 14) have an average surface area between 0.03 mm² and 5.0 mm².

5. Test carrier according to claim 1, characterised in that the total area of the melt adhesive regions (7, 13, 14) amounts to between 10% and 70%, preferably between 15% and 50% of the total connecting surface of the layers.

6. Test carrier according to claim 1, characterised in that it has a carrier layer (2) and several zones (3, 4) arranged thereon next to one another and that at least one of the zones is a liquid transport zone (3) in which both layers (2, 5) bound together by the melt adhesive regions (7) are not absorbent and that at least one of the layers (6) bound with one another by the melt adhesive regions (7) is absorbent.

7. Process for the production of a test carrier according to one of claims 1 to 6, characterised in that the melt adhesive in the form of discrete regions, between which intermediate spaces are present, is applied to a first layer material in a thickness which is greater than the desired gap and a second layer material is pressed against the melt adhesive regions while the melt adhesive is still sufficiently hot in order to be bindable in such a manner that it binds with the melt adhesive but a distance remains betwen the layer materials determined by the thickness of the solidified melt adhesive regions.

8. Process according to claim 7, characterised in that the pressing on of the second layer material takes place so shortly after the application of the melt adhesive to the first layer material that this is still sufficiently hot in order to be bindable.

9. Process according to claim 8, characterised in that the second layer material is provided with a layer containing a wetting agent.

10. Process according to claim 7, characterised in that the pressing on of the second layer material takes place with the help of two rollers between which the layer materials run, whereby the distance is determined by the distance of the rollers.

11. Process according to claim 7, characterised in that the melt adhesive regions are applied by a screen printing process.

12. Process according to claim 7, characterised in that the melt adhesive regions are applied with a wheel application device.

## Revendications

1. Support de test de diagnostic comportant au moins deux couches (2,5;2,6) qui sont reliées entre elles avec un certain écartement, de sorte qu'il existe entre elles une fente (8), caractérisé en ce que les couches (2,5;2,6) sont reliées entre elles par l'intermédiaire d'une pluralité de zones discrètes de colle à fusion (7,13,14), l'épaisseur des zones discrètes de colle à fusion (7,13,14) étant déterminante pour la fente (8), les zones de colle à fusion sont réparties sur la surface de jonction entre les couches (2,5;2,6), plusieurs interstices (9) existent entre les zones de colle à fusion, et un transport de liquide est possible dans la fente (8) à travers les interstices (9) entre les zones de colle à fusion (7,13,14).

2. Support de test de diagnostic selon la revendication 1, caractérisé en ce que les zones de colle à fusion (7,13,14) ont une épaisseur de 0,02 à 1,0 mm, de préférence 0,02 à 0,3 mm.

3. Support de test selon la revendication 1, caractérisé en ce que les zones sont des particules (7,14).

4. Support de test selon la revendication 3, caractérisé en ce que les particules (7,14) ont une surface moyenne de base comprise entre 0,03 mm² et 5,0 mm².

5. Support de test selon la revendication 1, caractérisé en ce que la surface totale des zones de colle à fusion (7,13,14) occupe entre 10 % et 70 %, de préférence entre 15 % et 50 % de la surface de jonction totale des couches.

6. Support de test selon la revendication 1, caractérisé en ce qu'il présente une couche de support (2) et plusieurs zones (3,4) disposées sur celle-ci de façon adjacente, et qu'au moins l'une des zones est une zone (3) de transport de liquide, dans laquelle les deux couches (2,5), reliées entre elles par l'intermédiaire de la zone de colle à fusion (7), ne sont pas absorbantes, et qu'au moins une autre zone est une zone d'évaluation (4), dans laquelle au moins l'une des couches (6), reliées entre elles par l'intermédiaire de zones de colle à fusion (7), est absorbante.

7. Procédé de préparation d'un support de test selon l'une des revendications 1-6, caractérisé en ce que la colle à fusion est appliquée sous la forme de zones discrètes, entre lesquelles se trouvent des interstices, sur une première substance de couche, en une épaisseur qui est supérieure à la fente voulue, et qu'une deuxième substance de couche est pressée contre les zones de colle à fusion pendant que la colle à fusion est suffisamment chaude, de telle façon qu'elle se lie à la colle à fusion, mais qu'entre les substances de couche, il reste toutefois une distance déterminée par l'épaisseur des zones de colle à fusion durcies.

8. Procédé selon la revendication 7, caractérisé en ce que la compression de la seconde substance de couche est effectuée suffisamment vite après l'application des zones de colle à fusion sur la première substance de couche pour que celle-ci soit encore suffisamment chaude et apte à se lier.

9. Procédé selon la revendication 8, caractérisé en ce que la seconde substance de couche est munie d'une couche contenant un agent mouillant.

10. Procédé selon la revendication 7, caractérisé en ce que la compression de la seconde substance de couche est effectuée à l'aide de deux cylindres entre lesquels passent les substances de couches, leur écartement étant déterminé par l'écartement des cylindres.

11. Procédé selon la revendication 7, caractérisé en ce que les zones de colle à fusion sont appliquées par un procédé de sérigraphie.

12. Procédé selon la revendication 7, caractérisé en ce que les zones de colle à fusion sont appliquées à l'aide d'un dispositif d'application à roue.
